# EUROPEAN PATENT SPECIFICATION

(11) **EP 1 827 303 B1**
(45) Date of publication and mention of the grant of the patent: **02.06.2010**
(21) Application number: 05810559.4
(22) Date of filing: 06.09.2005
(51) Int. Cl.: A61F 2/06, A61L 31/02, A61L 27/00

(54) **VULNERABLE PLAQUE STENT**
STENT FÜR VULNERABLE PLAQUE
STENT POUR PLAQUE VULNERABLE

(30) Priority: 22.12.2004 US 20537
(43) Date of publication of application: 05.09.2007
(73) Proprietor: Boston Scientific Limited, Christ Church (BB)
(72) Inventor: HOLMAN, Thomas, Princeton, MN 55371 (US); WEBER, Jan, Maple Grove, MN 55311 (US); KVEEN, Graig, Maple Grove, MN 55311 (US)
(74) Representative: Schildberg, Peter
(86) International application number: PCT/US2005/031690
(87) International publication number: WO 2006/071297

(56) References cited:
- WO-A-01/68158
- WO-A-02/24247
- WO-A-02/43788
- WO-A-03/026532
- WO-A-2005/046749
- WO-A-2005/077430
- WO-A2-03/028522

## Description

The body includes various passageways such as arteries, other blood vessels, and other body lumens. These passageways sometimes become occluded or weakened. For example, the passageways can be occluded by a tumor, restricted by plaque, or weakened by an aneurysm. When this occurs, the passageway can be reopened or reinforced, or even replaced, with a medical endoprosthesis. An endoprosthesis is typically a tubular member that is placed in a lumen in the body. Examples of endoprostheses include but are not limited to stents and covered stents, sometimes called 'stent-grafts' and other expandable, tubular frameworks.

Endoprostheses can be delivered inside the body in a compacted or reduced-size form by a catheter. Upon reaching the site, the endoprosthesis is expanded, for example, so that it can contact the walls of the lumen.

In one delivery technique, the endoprosthesis is formed of a material that can be reversibly compacted and expanded, e.g., elastically or through a material phase transition. During introduction into the body, the endoprosthesis is restrained in a compacted condition. Upon reaching the desired implantation site, the restraint is removed, for example, by retracting a restraining device such as an outer sheath, enabling the endoprosthesis to self expand by its own internal restoring force.

In another technique, a force may be applied to the endoprosthesis to expand it radially. For example, the catheter may carry a balloon, which carries a balloon-expandable endoprosthesis. The balloon can be inflated to deform and to fix the endoprosthesis at a predetermined position in contact with the lumen wall. The balloon can then be deflated, and the catheter withdrawn.

In some cases, the stent is to be positioned at a location in which vulnerable plaque (i.e. a lipid pool) is residing in the artery wall. If the vulnerable plaque is not treated, problems may arise when the wall allows the plaque to be released into the blood stream. This release may result in instant clotting of the vascular system at the location of the vulnerable plaque as well as instant clotting downstream from the location the plaque was released thereby reducing or completely stopping the flow of blood. In treating such a portion of the artery wall, care must betaken in order to avoid any rupture due to the pressure exerted by the stent or delivery system on the vulnerable plaque. Especially during placement of the stent rotational movement of the unfolding stent due to the unfolding balloon might result in high circumferential oriented shear forces. Stents typically provide a uniform radial pressure in all directions; this pressure may cause a rupture in the area of the plaque, as might the combination of radial pressure and shear forces.

WO 02/24247 discloses a covered stent having a framework of interconnected elongated members to the form of a hollow tube. A cover is disposed over a portion of the stent, either on the inside surface, the outside surface or intermediate the surfaces.

WO 03/026532 discloses medical devices including one or more components comprising one or more nanoparticles and/or nanocomposite materials.

WO 03/028522 discloses a flow reducing stent with expandable and non-expendable cells.

It would be desirable to both provide an improved endoluminal device which would exert less pressure on the vulnerable plaque while exerting radial pressure sufficient to maintain the fixation of the endoluminal device within the lumen and to reduce the or eliminate the shear forces that the stent is exerting on the membrane of the vulnerable plague.

Without limiting the scope of the invention, a brief summary of the claimed embodiments of the invention is set forth below. Additional details of the summarized embodiments of the invention and/or additional embodiments of the invention may be found in the detailed description of the invention and in the set of claims below.

### SUMMARY

The above problem is solved by the subject-matter of independent claim 1.

In at least one embodiment of the invention, a stent comprises a framework with a plurality of expandable and non-expandable openings therethrough and carbon nanotube sheet or "bucky paper". The carbon nanotube sheet may extend across at least one of the openings or across a plurality of openings in the framework. The carbon nanotube sheet may be disposed within the interior of the framework and/or about the exterior of the framework and/or between exterior and interior of the framework.

In at least one embodiment of the invention, the framework of the stent may include a plurality of interconnected struts, where the struts form a more rigid portion of the framework and a less rigid portion of the framework. The carbon nanotube sheet may extend across at least a portion of the more rigid portion.

In at least one embodiment of the invention, the framework of the stent includes a plurality of interconnected cells including expandable cells and non-expandable cells, wherein the carbon nanotube sheet may extend across one or more non-expandable cells. Though maintaining the substantially same shape throughout delivery, the non-expandable cells may be capable of flexing when the expandable portions of the framework of the stent expand from an unexpanded state to an expanded state.

The carbon nanotube sheet may be applied to the framework along the periphery of the carbon nanotube sheet and/or along other regions along the carbon nanotube sheet.

In at least one embodiment of the invention, the carbon nanotube sheet may be between portions of the framework.

In at least one embodiment of the invention, the framework may have a distal end and a proximal end wherein the distal end and the proximal end may be uniformly expandable about the circumference of the framework. Desirably, the ends will be capable of exerting a substantially uniform radial pressure. In the case of non-uniform vessels, for example vessels of non-uniform diameter, the stent will desirably exert different radial outward forces at the two ends of the stent.

In at least one embodiment of the invention, a therapeutic agent may be applied to the stent or the carbon canotube sheet. The agent may be on the surface of the stent and/or within the stent framework. In at least one embodiment, the carbon nanotube has strips of polymer which have a therapeutic agent within them.

In at least one embodiment of the invention, a SIBS sheet (e.g. polystyrene-polyisobutylene-polystyrene) may be used in affixing at least one carbon nanotube sheet to the framework. The SIBS sheet may be on the interior of the stent between the stent framework and a carbon nanotube sheet. In at least one embodiment the SIBS sheet may be on the stent framework and between two carbon nanotube sheets.

In at least one embodiment of the invention, at least one carbon nanotube sheet may be affixed to the framework by annealing. In at least one embodiment, a carbon nanotube sheet may be affixed to the framework by toluene treatment. In at least one embodiment, a carbon nanotube sheet may be affixed to a polymer framework by toluene treatment.

In at least one embodiment of the invention, at least one carbon nanotube sheet may be sprayed onto the exterior of the framework. In at least one embodiment, carbon nanotubes in solution are sprayed. In at least one embodiment, carbon nanotubes in a solution comprising tetrahydrofuran THFare sprayed onto the exterior of the framework. In at least one embodiment, a carbon nanotube sheet may be affixed to the interior of the framework in order to provide an additional backing for at least one carbon nanotube sheet being sprayed on the exterior of the stent framework.

In at least one embodiment, a sheet of carbon nanotube is affixed to the framework by brushing on a solution comprising carbon nanotubes which then dries onto the framework. In at least one embodiment, a sheet of carbon nanotube is affixed to the framework by dipping the framework into a solution comprising carbon nanotubes which then dries onto the framework. In at least one embodiment, a sheet of carbon nanotube is affixed to the framework by microdropping a solution comprising carbon nanotubes which then dries onto the framework.

In at least one embodiment of the invention, the stent may be selected from the group consisting of self-expanding stents, balloon expandable stents, hybrid stents, polymer stents, and degradable stents. In at least one embodiment, the stent may have a single backbone running along the length of the stent, thereby forming the basic scaffold of the stent design (henceforth referred to as a backbone stent); the backbone may have affixed to it a strip of carbon nanotube.

In at least one embodiment of the invention, at least one carbon nanotube sheet may cover at least one opening. In at least one embodiment of the invention, expansion of the at least one opening may be restricted by at least one carbon nanotube sheet which covers the at least one opening.

In at least one embodiment of the invention, at least one of the non-expandable cells may be constructed of ceramic or ploymer.

In at least one embodiment of the invention, the stent may comprise a rolled framework, the carbon nanotube sheet covering openings which are non-expanding.

Also disclosed is an expandable tubular insert for a bodily passage comprising a plurality of interconnected cells having openings therethrough. The insert includes expandable cells and cells which are non-expandable. Two or more of the non-expandable cells have one or more sidewalls in common. Desirably, one or more of the non-expandable cells have a maximum longitudinal length which is at least 50% of the maximum longitudinal length of at least one of the expandable cells. More desirably, one or more of the non-expandable cells have a maximum longitudinal length which is at least 75% of the maximum longitudinal length of at least one of the expandable cells. Most desirably, one or more of the non-expandable cells have a maximum longitudinal length which is substantially equal to the maximum longitudinal length of at least one of the expandable cells. The term "substantially equal to" is intended to include variations of up to 5%.

Desirably, the non-expandable cells are not located at either the proximal or distal end of the stent.

In at least one embodiment of the invention, the tubular insert may further comprise carbon nanotube sheet extending across at least one of the openings in the non-expandable cells.

The devices may be disposed about a delivery device such as a catheter. The device may optionally be configured to release a therapeutic agent. In use, the therapeutic agent will desirably be delivered in the region of the carbon nanotube sheet.

### DESCRIPTION of DRAWINGS

Figs. 1-2 are flat views of a stent with one portion having a carbon nanotube sheet.
Fig. 3 is a flat view of a stent having more rigid portions which may be covered with a carbon nanotube sheet.
Fig. 4 is a flat view of a stent with the expandable portion having a carbon nanotube sheet.
Fig. 5 is a perspective view of a stent with a carbon nanotube sheet portion.
Fig. 6a is a cross-sectional view of a stent in an unexpanded state with a carbon nanotube sheet applied.
Fig. 6b is a cross-sectional view of a stent in an expanded state with a carbon nanotube sheet applied.
Fig. 7a is a cross-sectional view of a rolled stent in an unexpanded state with a carbon nanotube sheet applied.
Fig. 7b is a cross-sectional view of a rolled stent in an expanded state with a carbon nanotube sheet applied.
Fig. 8a is a cross-sectional view of a stent with polymer strips applied to the carbon nanotube sheet.
Fig. 8b is a cross-sectional view of a stent with polymer strips within the carbon nanotube sheet.
Fig. 9 is a cross-sectional view of a stent with a SIBS sheet used in applying carbon nanotube sheets.
Fig. 10 is a cross-sectional view of a stent with two carbon nanotube sheets applied.
Fig. 11 is a cross-sectional view of a stent with a carbon nanotube sheet applied and another carbon nanotube sheet which is sprayed on.
Fig. 12 is a cross-sectional view of a stent and delivery device within a lumen showing the stent being delivered and therapeutic agent applied.
Fig. 13 is a cross-sectional view of a stent and delivery device within a lumen showing the delivered stent and the delivered therapeutic agent.

### DETAILED DESCRIPTION

While this invention may be embodied in many different forms, there are described in detail herein specific embodiments of the invention. This description is an exemplification of the principles of the invention.

For the purposes of this disclosure, like reference numerals in the figures shall refer to like features unless otherwise indicated.

Also for the purposes of this disclosure, the term 'endoprosthesis' refers to an expandable prosthesis for implantation into a body lumen or vessel and includes devices such as stents, grafts, stent-grafts, vena cava filters, tubular expandable frameworks (regardless of whether they can support a vessel), etc.

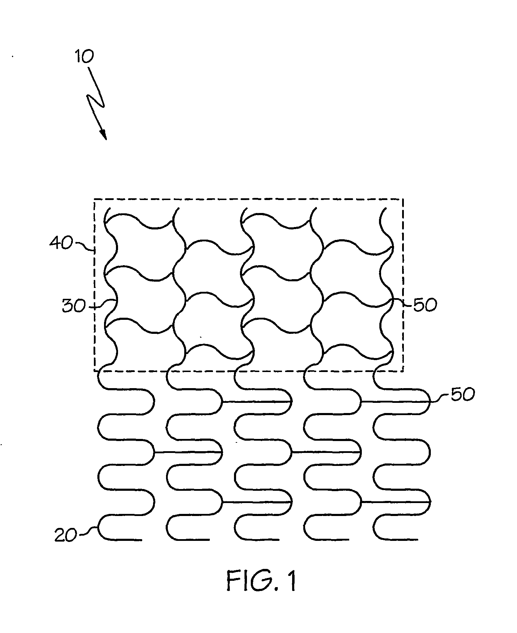

Referring to Fig. 1, a stent 10 is shown generally having an expandable portion 20 and a non-expandable portion 30. The non-expandable portion 30 may be flexible longitudinally. The expandable portion 20 may have portions that smoothly transition into the non-expandable portion 30. In some embodiments this transition is gradual. This can occur when, for example, there is a gradual change in flexibility over a desired region. The change in flexibility can be accomplished by, for example, a change in framework geometry and/or a change in some other framework parameter(s) such as thickness of the struts, width of the struts or combinations thereof. In some embodiments the transition is abrupt, for instance as when an expandable portion 20 and a non-expandable portion 30 are welded together. The non-expandable portion 30 may have less structure than the expandable portion 20.

The non-expandable portion has one or more carbon nanotube sheets 40 applied to it. The carbon nanotube sheet 40 may disposed on the interior of the framework and/or on the exterior of the framework. The carbon nanotube sheet desirably will allow red blood cells through to feed the tissue but will be impervious to lipids. Typically, the carbon nanotube sheet will be provided only in the less flexible or non-flexible portion of the framework. Desirably, the carbon nanotube sheet will be operatively associated with a portion of a framework which will be used to cover a lipid pool such that lipids are not released from beneath the carbon nanotube sheet. In some embodiments, the carbon nanotube sheet 40 may only be attached on its periphery to the framework 50 of the stent 10.

Carbon nanotube is described in greater detail in US 20040073251.

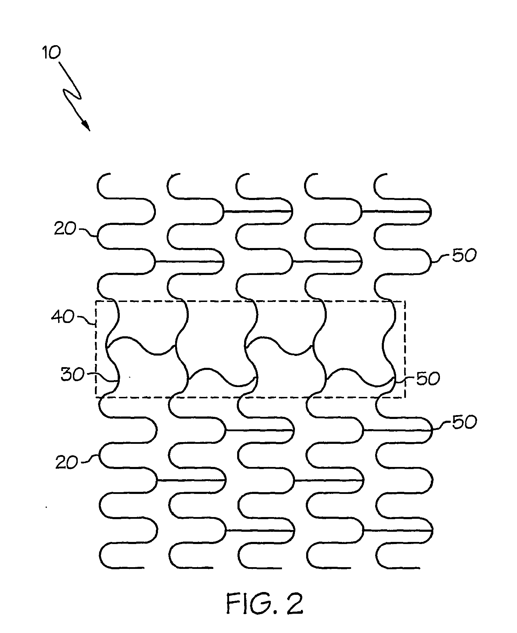

In Fig. 2, the non-expandable portion 30 has less structure than in Fig. 1. Thus, the amount of stent material (e.g. metal or polymeric material) per unit area is less in the non-expandable portion than in the expandable portion. In some embodiments, the carbon nanotube sheet 40 may be supported only on its periphery by framework 50 of the stent 10.

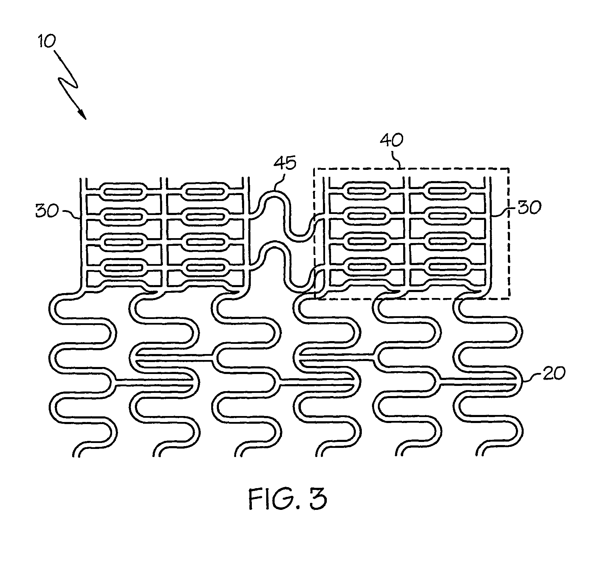

As shown in Fig. 3 there may be more than one non-expandable portion 30. At least one connector 45 may be disposed longitudinally between the non-expandable portions 30. The connector may have one or more curves or may be straight. The connector may be parallel to the longitudinal axis or may extend at a non-zero angle relative to the longitudinal axis. The ends of the connector may lie along a line parallel to the longitudinal axis of the stent. It is also within the scope of the invention for the ends of the connector to lie along two separate lines parallel to the longitudinal axis of the stent. In such a case, the ends of the connectors are not considered to be aligned circumferentially. It is also with the scope of the invention for there to be a plurality of non-expandable portions which are separated from one another by expandable portions. Some or all of the non-expanding portions 30 may be rigid and/or may have a greater concentration of structure than the expandable portions 20.

A carbon nanotube sheet is applied to one or more of the non-expandable portions 30. In some embodiments a single sheet of carbon nanotube covers more than one non-expandable portion 30 and the at least one connector 45 between the two non-expandable portions.

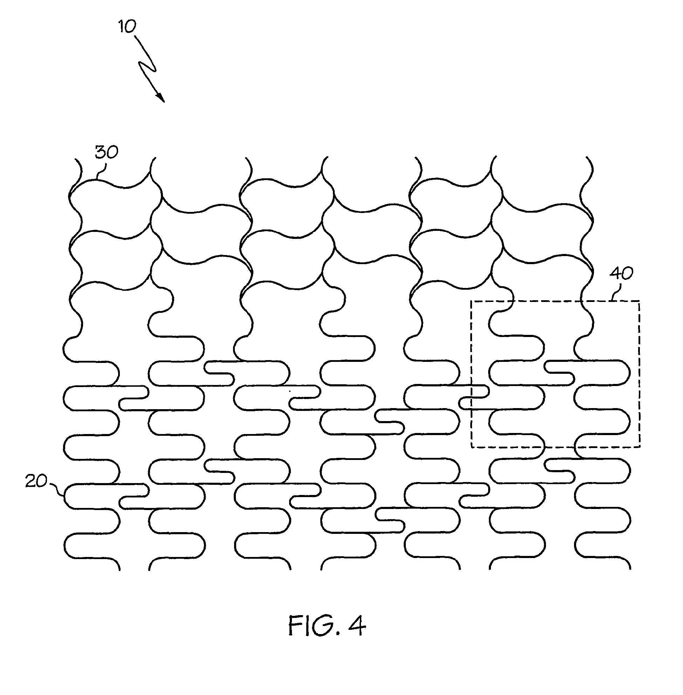

In some embodiments, as illustrated in Fig. 4, the carbon nanotube sheet may be applied to at least a portion of the expandable portion 40 of the stent 10. In some embodiments, the carbon nanotube sheet 40 may be applied to both the non-expandable portion(s) 30 and the expandable portion(s) 20. Desirably, particularly in the case where the carbon nanotube sheet is provided in the expandable portion(s), the carbon nanotube sheet may expand or unfold from a folded condition.

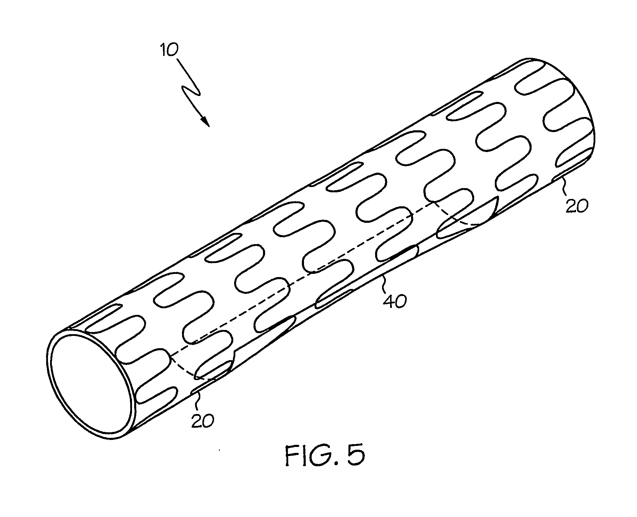

The region with carbon nanotube sheet may be at one or both ends of the stent or may be disposed between the ends of the stent, as illustrated in Fig. 5. Stent 10, shown in Fig. 5, includes a patch of carbon nanotube sheet 40 between the ends of the stent and extending over a portion of the circumference of the framework of the stent but not over the entirety of the circumference of the framework.

Any of the embodiments disclosed herein may be provided in self-expanding, balloon expandable, or hybrid designs. In the case of self-expanding stents, the portion of the stent having the carbon nanotube sheet patch 40 may not exert as much radial pressure as the other portions of the stent 10.

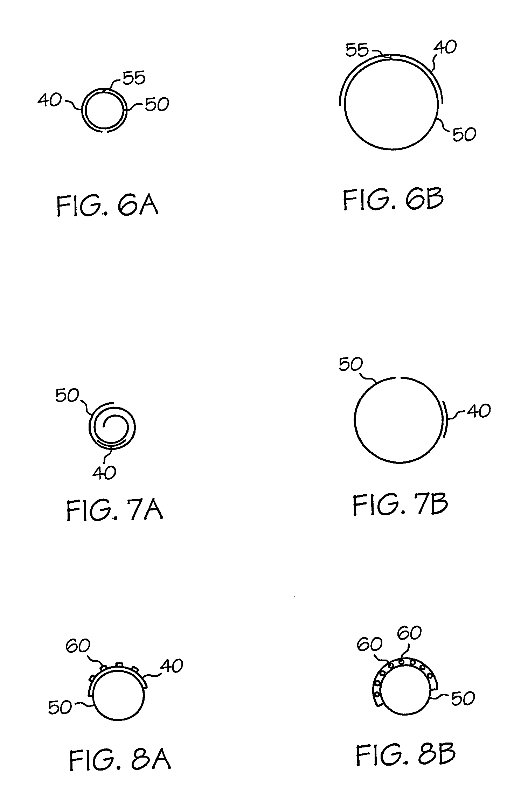

In Fig. 6a, a stent is shown in the unexpanded state having a carbon nanotube sheet 40. The sheet 40 may be attached to the stent framework 50 at a single connection point 55. The stent framework may be in the form of a backbone stent wherein the connection point 55 is located on the "backbone" of the framework 50. The unattached portions of sheet 40 may freely glide along the framework 50 such that when the stent framework 50 is expanded, as shown in Fig. 6b, the sheet 40 moves with the expansion without restricting the expansion.

Fig. 7a illustrates a rolled stent framework 50 in the unexpanded state having a carbon nanotube sheet 40 applied. In the expanded state shown in Fig. 7b, the rolled stent framework 50 unrolls while the sheet 40 continues to cover the same portion of the framework 50 covered in the unexpanded state.

A therapeutic agent may be applied to the carbon nanotube sheet 40 as shown in Figs. 8a and 8b. Polymer strips 60 containing the therapeutic agent may be applied to the surface of the carbon nanotube sheet 40 or within the sheet 40.

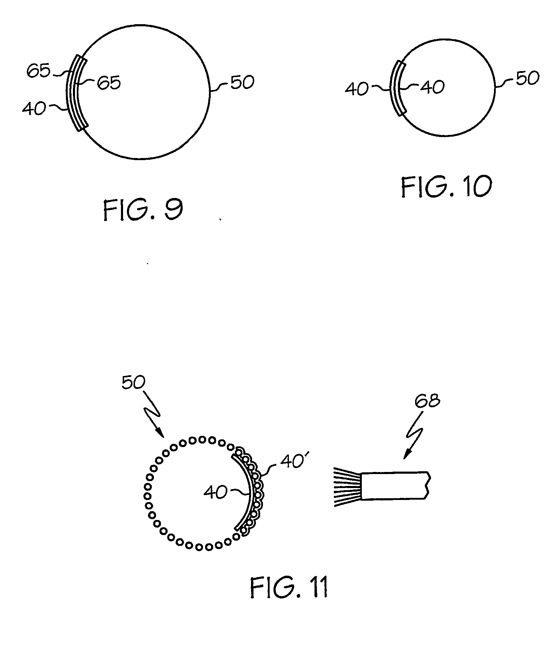

As shown in Fig. 9 a SIBS sheet 65 may be used in applying one or more carbon nanotube sheets 40 to the stent framework 50. Fig. 10 illustrates two carbon nanotube sheets 40 applied to the stent framework 50. The sheets 40 may be affixed to the framework 50 through annealing, or through toluene treatment in the case of a polymer framework 50.

A carbon nanotube sheet may also be applied to the stent framework 50 by spraying on the sheet 40' by a sprayer 68 as shown in Fig. 11. An additional carbon nanotube sheet 40 may be applied to the framework 50 before the sprayed on sheet 50 is applied. In some embodiments the additional sheet 40 is not applied.

In some embodiments the stent framework may degrade within the body lumen such that only the carbon nanotube sheet remains at the treated site.

Vessels can be stented using stents comprising carbon nanotube sheet.

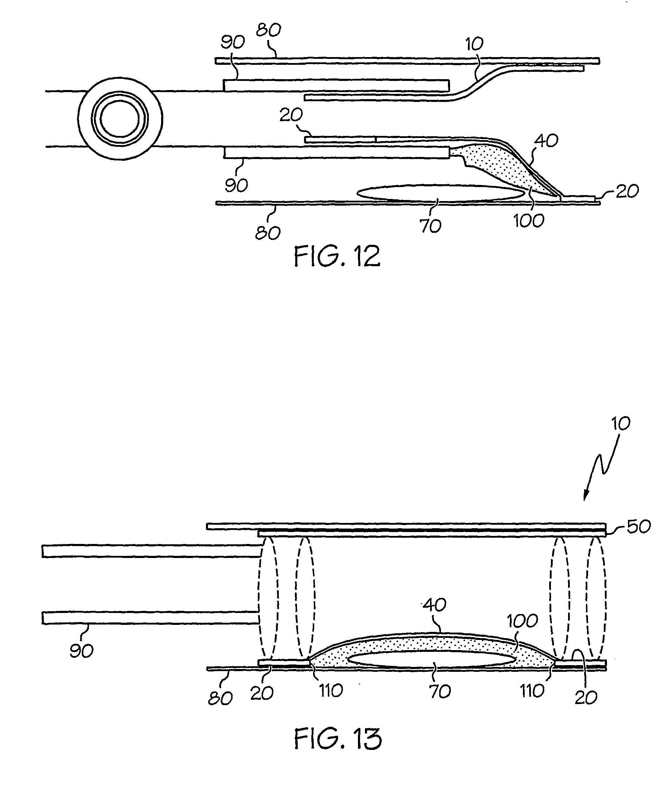

In Fig. 12, a stent is delivered to a portion of a body lumen 80 having a lipid pool 70 or "vulnerable plaque". The delivery device 90 may release one end of the stent 10 distal to the lipid pool 70. The distal most end of the stent 10 may comprise an expandable portion which may expand and exert radial pressure on the lumen 80. The carbon nanotube sheet portion 40 may then be partially released and positioned such that, upon complete delivery of the stent 10, the carbon nanotube sheet portion 40 may be disposed upon the lipid pool 70. The carbon nanotube sheet portion 40 may exert less radial pressure than the expandable portions 20 of the stent 10 such that the stent may maintain the patency of the lumen 80 while at the same time covering the lipid pool portion 70 without rupturing it due to excessive radial pressure. As the stent 10 is being delivered, a therapeutic agent 100 may be released upon the lipid pool 70 such that upon full delivery the carbon nanotube sheet portion 40 may be disposed upon both the lipid pool 70 and the therapeutic agent 100.

Fig. 13 shows a stent 10 which has been deployed in a vessel with vulnerable plaque. The stent is held in position in lumen 80 because of the expandable portions 20 which have circumferential stiffness both proximally and distally of the lipid pool 70. The stent is aligned such that the carbon nanotube sheet covers the vulnerable plaque to prevent or hinder the release of lipids into the lumen 80. In addition, a seal may be created about a therapeutic agent 100 such that the therapeutic agent is spread onto the vulnerable plaque. In at least one embodiment, the stent framework 50 or other element of the device 10 may comprise one or more therapeutic agents. The agent may be placed on the stent in the form of a coating. The coating may include at least one therapeutic agent and at least one polymer agent.

A therapeutic agent may be a drug or other pharmaceutical product such as non-genetic agents, genetic agents, cellular material, etc. Some examples of suitable non-genetic therapeutic agents include but are not limited to: anti-thrombogenic agents such as heparin, heparin derivatives, vascular cell growth promoters, growth factor inhibitors, Paclitaxel, etc. Where an agent includes a genetic therapeutic agent, such a genetic agent may include but is not limited to: DNA, RNA and their respective derivatives and/or components; hedgehog proteins, etc. Where a therapeutic agent includes cellular material, the cellular material may include but is not limited to: cells of human origin and/or non-human origin as well as their respective components and/or derivatives thereof. Where the therapeutic agent includes a polymer agent, the polymer agent may be a polystyrene-polyisobutylene-polystyrene triblock copolymer (SIBS), polyethylene oxide, silicone rubber and/or any other suitable substrate.

The use of carbon nanotube sheet has been discussed above with respect to stents. More generally, it is within the scope of the invention to use carbon nanotube sheet with endoprostheses. The carbon nanotube sheet may be associated with the entirety of the endoprosthesis or with less than the entirety of the endoprosthesis. The endoprosthesis will include non-expandable cells and expandable cells and the carbon nanotube sheet will be disposed on or in one or more of the non-expandable cells, as is discussed specifically for stents.

Desirably, the portion of the inventive stents in particular and inventive endoprostheses in general that includes the carbon nanotube sheet will exert a lesser outward force as compared to those regions that do not include carbon nanotube sheet. This may be of particular importance where the portion of the device with the carbon nanotube sheet is disposed against a weaker region of a vessel or a region with vulnerable plaque.

The inventive stents and, more generally, endoprostheses may find use in coronary arteries, renal arteries, peripheral arteries including illiac arteries, arteries of the neck and cerebral arteries. The stents and, more generally, endoprostheses of the present invention, however, are not limited to use in the vascular system and may also be advantageously employed in other body structures, including but not limited to arteries, veins, biliary ducts, urethras, fallopian tubes, bronchial tubes, the trachea, the esophagus and the prostate.

The inventive stents and endoprostheses disclosed herein may be at least partially constructed of any of a variety of materials such as stainless steel, nickel, titanium, nitinol, platinum, gold, chrome, cobalt, as well as any other metals and their combinations or alloys. In some embodiments, the endoprosthesis may be at least partially constructed of a polymer material. In some embodiments, the endoprosthesis may be at least partially constructed of a shape-memory polymer or material. In some embodiments, the endoprosthesis may be balloon expandable, self-expandable, hybrid expandable or a combination thereof. The endoprosthesis may include one or more radiopaque members. The endoprosthesis may include one or more therapeutic and/or lubricious coatings applied thereto.

The above disclosure is intended to be illustrative and not exhaustive. This description will suggest many variations and alternatives to one of ordinary skill in this art. The various elements shown in the individual figures and described above may be combined or modified for combination as desired. All these alternatives and variations are intended to be included within the scope of the claims where the term "comprising" means "including, but not limited to". Those familiar with the art may recognize other equivalents to the specific embodiments described herein which equivalents are also intended to be encompassed by the claims.

## Claims

1. An endoprothesis (10) having a framework (50) with a plurality of openings therethrough and at least one sheet (40), the at least one sheet (40) extending across at least one of the openings, **characterized in that**
the plurality of openings comprises a plurality of expandable cells (20) and a plurality of non-expandable cells (30), wherein the at least one sheet (40) is a carbon nanotube sheet which extends over the non-expandable cells (30).

2. The endoprosthesis (10) of claim 1, in the form of a stent (10).

3. The endoprosthesis (10) of claim 1, the expandable framework (50) having an interior and an exterior, wherein the at least one carbon nanotube sheet (40) is disposed within the interior of the expandable framework (50).

4. The endoprosthesis (10) of claim 1, the expandable framework (50) having an interior and an exterior, wherein the at least one carbon nanotube sheet (40) is disposed about the exterior of the expandable framework (50).

5. The endoprosthesis (10) of claim 2 wherein the framework (50) includes a plurality of interconnected struts, the struts forming a more rigid portion of the framework (50) and a less rigid portion of the framework (50), the at least one carbon nanotube sheet (40) extending across at least a portion of the more rigid portion.

6. The endoprosthesis (10) of claim 2 wherein the at least one carbon nanotube sheet (40) has a peripheral region, the at least one carbon nanotube sheet (40) applied to the framework solely at the peripheral region.

7. The endoprosthesis (10) of claim 2 wherein the at least one carbon nanotube sheet (40) is between portions of the framework (50).

8. The endoprosthesis (10) of claim 2 wherein the expandable framework (50) has a distal end and a proximal end, the distal end and proximal end being uniformly expandable about the circumference of the stent.

9. The endoprosthesis (10) of claim 2 further comprising a therapeutic agent.

10. The endoprosthesis (10) of claim 2 wherein a SIBS sheet (65) is used in affixing at least one carbon nanotube sheet (40) to the framework (50).

11. The endoprosthesis (10) of claim 10 having multiple carbon nanotube sheets (40), the SIBS sheet (65) being disposed between carbon nanotube sheets (40).

12. The endoprosthesis (10) of claim 2 wherein at least one carbon nanotube sheet is applied to the framework (50) by annealing or by treatment of a solvent.

13. The endoprosthesis (10) of claim 12 wherein the solvent is inorganic.

14. The endoprosthesis (10) of claim 12 wherein the solvent is organic.

15. The endoprosthesis (10) of claim 12 wherein the solvent is selected from the group consisting of toluene, THF, DNA, gum Arabic, or any combination thereof.

16. The endoprosthesis (10) of claim 2 wherein at least one carbon nanotube sheet is sprayed onto the exterior of the framework (50).

17. The endoprosthesis (10) of claim 3 wherein at least one carbon nanotube sheet is sprayed onto the exterior of the framework (50).

18. The endoprosthesis (10) of claim 2 wherein the stent (10) is selected from the group consisting of self-expanding stents, balloon expandable stents, and hybrid stents.

19. The endoprosthesis (10) of claim 2 wherein the stent (10) is a polymer stent.

20. The endoprosthesis (10) of claim 2 wherein the stent (10) is a degradable stent.

21. The endoprosthesis (10) of claim 1 wherein the carbon nanotube sheet (40) is a stripe along the stent (10).

22. The endoprosthesis (10) of claim 2 wherein at least one carbon nanotube sheet covers at least one cell, expansion of the at least one cell restricted by the at least one carbon nanotube sheet (40) which covers the at least one cell.

23. The endoprosthesis (10) of claim 1 wherein at least one of the non-expandable cells is constructed of ceramic.

24. The endoprosthesis (10) of claim 2 wherein the expandable framework (50) is a rolled expandable framework, the carbon nanotube sheet (40) covering cells which are non-expanding (30).

25. The endoprosthesis (10) of claim 2 wherein the framework (50) has a distal end and a proximal end, the distal end and the proximal end are constructed and arranged to exert a substantially uniform radial pressure.

26. The endoprosthesis (10) of claim 1 wherein the at least one carbon nanotube sheet (40) extends over only a portion of the framework (50) between a proximal and distal end of the framework (50).

27. The endoprosthesis (10) of claim 1 wherein the framework (50) includes a plurality of interconnected struts, the struts forming a more rigid portion of the framework and a less rigid portion of the framework, the at least one carbon nanotube sheet (40) extending across at least a portion of the more rigid portion.

## Patentansprüche

1. Endoprothese (10), die ein Rahmenwerk (50) mit einer Mehrzahl Öffnungen **dadurch** und mindestens ein Blech (40) aufweist, wobei sich das mindestens eine Blech (40) über mindestens eine der Öffnungen erstreckt, **dadurch gekennzeichnet, dass**
die Mehrzahl Öffnungen eine Mehrzahl expandierbarer Zellen (20) und eine Mehrzahl nicht expandierbarer Zellen (30) umfasst, wobei das mindestens eine Blech (40) ein Kohlenstoff-Nanoröhrchen-Blech ist, das sich über die nicht expandierbaren Zellen (30) erstreckt.

2. Endoprothese (10) nach Anspruch 1, in Form eines Stents (10).

3. Endoprothese (10) nach Anspruch 1, wobei das expandierbare Rahmenwerk (50) ein Inneres und ein Äußeres aufweist, wobei das mindestens eine Kohlenstoff-Nanoröhrchen-Blech (40) innerhalb des Inneren des expandierbaren Rahmenwerks (50) angeordnet ist.

4. Endoprothese (10) nach Anspruch 1, wobei das expandierbare Rahmenwerk (50) ein Inneres und ein Äußeres aufweist, wobei das mindestens eine Kohlenstoff-Nanoröhrchen-Blech (40) um das Äußere des expandierbaren Rahmenwerks (50) angeordnet ist.

5. Endoprothese (10) nach Anspruch 2, wobei das Rahmenwerk (50) eine Mehrzahl miteinander verbundener Streben umfasst, wobei die Streben einen steiferen Abschnitt des Rahmenwerks (50) und einen weniger steifen Abschnitt des Rahmenwerks (50) bilden, wobei sich das mindestens eine Kohlenstoff-Nanoröhrchen-Blech (40) über mindestens einen Abschnitt des steiferen Abschnitts erstreckt.

6. Endoprothese (10) nach Anspruch 2, wobei das mindestens eine Kohlenstoff-Nanoröhrchen-Blech (40) einen Randbereich aufweist, wobei das mindestens eine Kohlenstoff Nanoröhrchen-Blech (40) nur an dem Randbereich an dem Rahmenwerk angebracht ist.

7. Endoprothese (10) nach Anspruch 2, wobei das mindestens eine Kohlenstoff-Nanoröhrchen-Blech (40) zwischen Abschnitten des Rahmenwerks (50) liegt.

8. Endoprothese (10) nach Anspruch 2, wobei das expandierbare Rahmenwerk (50) ein distales Ende und ein proximales Ende aufweist, wobei das distale Ende und das proximale Ende gleichförmig um den Umfang des Stents expandierbar sind.

9. Endoprothese (10) nach Anspruch 2, überdies umfassend einen therapeutischen Wirkstoff.

10. Endoprothese (10) nach Anspruch 2, wobei ein SIBS-Blech (65) beim Befestigen von mindestens einem Kohlenstoff-Nanoröhrchen-Blech (40) an dem Rahmenwerk (50) verwendet wird.

11. Endoprothese (10) nach Anspruch 10, die mehrfache Kohlenstoff-Nanoröhrchen-Bleche (40) aufweist, wobei das SIBS-Blech (65) zwischen Kohlenstoff-Nanoröhrchen-Blechen (40) angeordnet ist.

12. Endoprothese (10) nach Anspruch 2, wobei mindestens ein Kohlenstoff-Nanoröhrchen-Blech an dem Rahmenwerk (50) durch Glühen oder Lösungsmittelbehandlung angebracht wird.

13. Endoprothese (10) nach Anspruch 12, wobei das Lösungsmittel anorganisch ist.

14. Endoprothese (10) nach Anspruch 12, wobei das Lösungsmittel organisch ist.

15. Endoprothese (10) nach Anspruch 12, wobei das Lösungsmittel aus der Gruppe ausgewählt ist, die aus Toluen, THF, DNS, Gummiarabikum oder einer beliebigen Kombination davon besteht.

16. Endoprothese (10) nach Anspruch 2, wobei mindestens ein Kohlenstoff-Nanoröhrchen-Blech auf das Äußere des Rahmenwerks (50) gesprüht ist.

17. Endoprothese (10) nach Anspruch 3, wobei mindestens ein Kohlenstoff-Nanoröhrchen-Blech auf das Äußere des Rahmenwerks (50) gesprüht ist.

18. Endoprothese (10) nach Anspruch 2, wobei der Stent (10) aus der Gruppe ausgewählt ist, die aus selbstexpandierbaren Stents, ballonexpandierbaren Stents und Hybridstents besteht.

19. Endoprothese (10) nach Anspruch 2, wobei der Stent (10) ein Polymerstent ist.

20. Endoprothese (10) nach Anspruch 2, wobei der Stent (10) ein abbaubarer Stent ist.

21. Endoprothese (10) nach Anspruch 1, wobei das Kohlenstoff-Nanoröhrchen-Blech (40) ein Streifen entlang dem Stent (10) ist.

22. Endoprothese (10) nach Anspruch 2, wobei mindestens ein Kohlenstoff-Nanoröhrchen-Blech mindestens eine Zelle bedeckt, wobei die Expansion der mindestens einen Zelle durch das mindestens eine Kohlenstoff-Nanoröhrchen-Blech (40), das die mindestens eine Zelle bedeckt, beschränkt ist.

23. Endoprothese (10) nach Anspruch 1, wobei mindestens eine der nicht expandierbaren Zellen aus Keramik aufgebaut ist.

24. Endoprothese (10) nach Anspruch 2, wobei das expandierbare Rahmenwerk (50) ein gerolltes expandierbares Rahmenwerk ist, wobei das Kohlenstoff-Nanoröhrchen-Blech (40) Zellen bedeckt, die nicht-expandierend (30) sind.

25. Endoprothese (10) nach Anspruch 2, wobei das Rahmenwerk (50) ein distales Ende und ein proximales Ende aufweist, wobei das distale Ende und das proximale Ende aufgebaut und angeordnet sind, um einen im Wesentlichen gleichförmigen radialen Druck auszuüben.

26. Endoprothese (10) nach Anspruch 1, wobei sich das mindestens eine Kohlenstoff-Nanoröhrchen-Blech (40) über nur einen Abschnitt des Rahmenwerks (50) zwischen einem proximalen und einem distalen Ende des Rahmenwerks (50) erstreckt.

27. Endoprothese (10) nach Anspruch 1, wobei das Rahmenwerk (50) eine Mehrzahl miteinander verbundener Streben umfasst, wobei die Streben einen steiferen Abschnitt des Rahmenwerks und einen weniger steifen Abschnitt des Rahmenwerks bilden, wobei sich das mindestens eine Kohlenstoff-Nanoröhrchen-Blech (40) über mindestens einen Abschnitt des steiferen Abschnitts erstreckt.

## Revendications

1. Endoprothèse (10) présentant une ossature (50) avec une multiplicité d'ouvertures à travers celle-ci et au moins une feuille (40), la au moins une feuille (40) s'étendant à travers au moins une des ouvertures, **caractérisée en ce que**
la multiplicité d'ouvertures comprend une multiplicité de cellules extensibles (20) et une multiplicité de cellules non extensibles (30), dans laquelle la au moins une feuille (40) est une feuille de nanotube de carbone qui s'étend sur les cellules non extensibles (30).

2. Endoprothèse (10) selon la revendication 1, sous la forme d'un stent (10).

3. Endoprothèse (10) selon la revendication 1, l'ossature extensible (50) présentant un intérieur et un extérieur, dans laquelle la au moins une feuille de nanotube de carbone (40) est disposée dans l'intérieur de l'ossature extensible (50).

4. Endoprothèse (10) selon la revendication 1, l'ossature extensible (50) présentant un intérieur et un extérieur, dans laquelle la au moins une feuille de nanotube de carbone (40) est disposée autour de l'extérieur de l'ossature extensible (50).

5. Endoprothèse (10) selon la revendication 2, dans laquelle l'ossature (50) comprend une multiplicité d'entretoises interconnectées, les entretoises formant une portion plus rigide de l'ossature (50) et une portion moins rigide de l'ossature (50), la au moins une feuille de nanotube de carbone (40) s'étendant à travers au moins une portion de la portion plus rigide.

6. Endoprothèse (10) selon la revendication 2, dans laquelle la au moins une feuille de nanotube de carbone (40) présente une région périphérique, la au moins une feuille de nanotube de carbone (40) étant appliquée à l'ossature uniquement à la région périphérique.

7. Endoprothèse (10) selon la revendication 2, dans laquelle la au moins une feuille de nanotube de carbone (40) est entre des portions de l'ossature (50).

8. Endoprothèse (10) selon la revendication 2, dans laquelle l'ossature extensible (50) présente une extrémité distale et une extrémité proximale, l'extrémité distale et l'extrémité proximale étant extensibles de manière uniforme autour de la circonférence du stent.

9. Endoprothèse (10) selon la revendication 2, comprenant en outre un agent thérapeutique.

10. Endoprothèse (10) selon la revendication 2, dans laquelle une feuille de SIBS (65) est utilisée lors de la fixation d'au moins une feuille de nanotube de carbone (40) sur l'ossature (50).

11. Endoprothèse (10) selon la revendication 10, présentant de multiples feuilles de nanotube de carbone (40), la feuille de SIBS (65) étant disposée entre des feuilles de nanotube de carbone (40).

12. Endoprothèse (10) selon la revendication 2, dans laquelle au moins une feuille de nanotube de carbone est appliquée sur l'ossature (50) par recuit ou par traitement d'un solvant.

13. Endoprothèse (10) selon la revendication 12, dans laquelle le solvant est inorganique.

14. Endoprothèse (10) selon la revendication 12, dans laquelle le solvant est organique.

15. Endoprothèse (10) selon la revendication 12, dans laquelle le solvant est sélectionné dans le groupe constitué du toluène, du THF, de l'ADN, de la gomme arabique, ou toute combinaison de ceux-ci.

16. Endoprothèse (10) selon la revendication 2, dans laquelle au moins une feuille de nanotube de carbone est pulvérisée sur l'extérieur de l'ossature (50).

17. Endoprothèse (10) selon la revendication 3, dans laquelle au moins une feuille de nanotube de carbone est pulvérisée sur l'extérieur de l'ossature (50).

18. Endoprothèse (10) selon la revendication 2, dans laquelle le stent (10) est sélectionné dans le groupe constitué des stents auto-extensibles, des stents extensibles à ballonnets, et des stents hybrides.

19. Endoprothèse (10) selon la revendication 2, dans laquelle le stent (10) est un stent polymère.

20. Endoprothèse (10) selon la revendication 2, dans laquelle le stent (10) est un stent dégradable.

21. Endoprothèse (10) selon la revendication 1, dans laquelle la feuille de nanotube de carbone (40) est une bande le long du stent (10).

22. Endoprothèse (10) selon la revendication 2, dans laquelle au moins une feuille de nanotube de carbone recouvre au moins une cellule, l'expansion de la au moins une cellule étant limitée par la au moins une feuille de nanotube de carbone (40) qui recouvre la au moins une cellule.

23. Endoprothèse (10) selon la revendication 1, dans laquelle au moins une des cellules non extensibles est constituée de céramique.

24. Endoprothèse (10) selon la revendication 2, dans laquelle l'ossature extensible (50) est une ossature extensible roulée, la feuille de nanotube de carbone (40) recouvrant des cellules ne s'étendant pas (30).

25. Endoprothèse (10) selon la revendication 2, dans laquelle l'ossature (50) présente une extrémité distale et une extrémité proximale, l'extrémité distale et l'extrémité proximale sont conçues et placées pour exercer une pression radiale essentiellement uniforme.

26. Endoprothèse (10) selon la revendication 1, dans laquelle la au moins une feuille de nanotube de carbone (40) s'étend sur seulement une portion de l'ossature (50) entre une extrémité proximale et une extrémité distale de l'ossature (50).

27. Endoprothèse (10) selon la revendication 1, dans laquelle l'ossature (50) comprend une multiplicité d'entretoises interconnectées, les entretoises formant une portion plus rigide de l'ossature et une portion moins rigide de l'ossature, la au moins une feuille de nanotube de carbone (40) s'étendant à travers au moins une portion de la portion plus rigide.
